(19) Europäisches Patentamt
European Patent Office
Office européen des brevets

(11) Veröffentlichungsnummer: 0 439 835 A2

# (12) EUROPÄISCHE PATENTANMELDUNG

(21) Anmeldenummer: 90125635.4

(22) Anmeldetag: 28.12.90

(51) Int. Cl.5: C07D 231/14, A01N 43/56, C07D 401/04, A01N 43/40

(30) Priorität: 20.01.90 DE 4001600

(43) Veröffentlichungstag der Anmeldung: 07.08.91 Patentblatt 91/32

(84) Benannte Vertragsstaaten: AT BE CH DE DK ES FR GB IT LI NL SE

(71) Anmelder: HOECHST AKTIENGESELLSCHAFT
Postfach 80 03 20
W-6230 Frankfurt am Main 80(DE)

(72) Erfinder: Lachhein, Stephen, Dr.
Kiedricher Strasse 18
W-6238 Hofheim am Taunus(DE)

## (54) Verfahren zur Herstellung von Pyrazolcarbonsäurederivaten.

(57) Verfahren zur Herstellung von Pyrazolcarbonsäurederivaten der Formel I

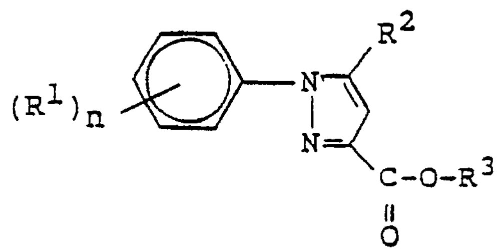

(I),

worin
$R^1$ = unabhängig voneinander Haloalkyl oder Halogen,
$R^2$ = Alkyl oder Alkinyl,
$R^3$ = Alkali- oder Erdalkalimetall, Wasserstoff, Alkyl oder Alkoxyalkyl und
n = 1 oder 2
bedeuten,
durch Umsetzung einer Verbindung der Formel II

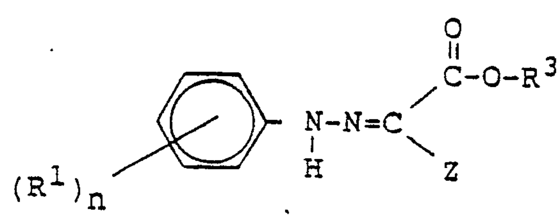

(II),

worin
Z für Chlor oder Brom steht,
mit einem Enolether der Formel III

EP 0 439 835 A2

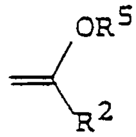

(III),

worin
$R^5$ = Alkyl ist, zu einer Verbindung der Formel IV

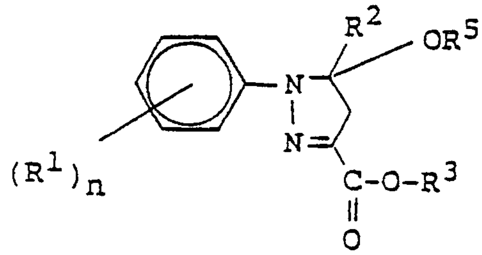

(IV),

und deren anschließender Aromatisierung zur Verbindung I, dadurch gekennzeichnet, daß man die Umsetzung der Verbindung II mit der Verbindung III zur Verbindung IV in einem 2-Phasensystem (wäßrig/organisch) in Gegenwart einer anorganischen Base bei Temperaturen von 40 - 100 °C und bei pH-Werten von 7 - 10 durchführt und anschließend die gebildete Verbindung IV ohne vorherige Isolierung unter sauren Bedingungen in einem 2-Phasensystem (wäßrig/organisch) zur Verbindung der Formel I aromatisiert.

## VERFAHREN ZUR HERSTELLUNG VON PYRAZOLCARBONSÄUREDERIVATEN

Gegenstand der vorliegenden Erfindung ist ein Verfahren zur Herstellung von Pyrazolcarbonsäurederivaten der Formel I

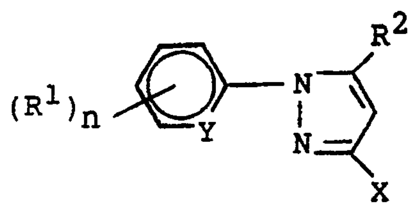

(I),

worin
Y
C-H oder N,
$R^1$
unabhängig voneinander ($C_1$-$C_4$)-Alkyl, ($C_1$-$C_4$)-Haloalkyl, ($C_1$-$C_4$)-Alkoxy, ($C_1$-$C_4$)-Haloalkoxy oder Halogen,
$R^2$
($C_1$-$C_{12}$)-Alkyl, ($C_3$-$C_7$)-Cycloalkyl, ($C_2$-$C_6$)-Alkenyl oder ($C_2$-$C_4$)-Alkinyl,
X
$COOR^3$, $CON(R^4)_2$, $COSR^3$ CN,

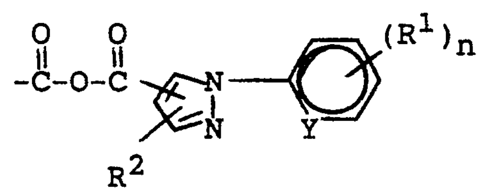

$R^3$
Alkali- oder Erdalkalimetall, Wasserstoff, ($C_1$-$C_{10}$)-Alkyl, ($C_3$-$C_{20}$)-Alkenyl, ($C_3$-$C_{10}$)-Alkinyl, ($C_3$-$C_7$)-Cycloalkyl, Phenyl-($C_1$-$C_4$)-Alkyl, wobei Phenyl durch Halogen substituiert sein kann, Tris-($C_1$-$C_4$)-Alkyl-Silyl-($C_1$-$C_4$)-alkyl, ($C_1$-$C_4$)-Alkoxy-($C_1$-$C_4$)-alkyl
$R^4$
unabhängig voneinander H, ($C_1$-$C_{10}$)-Alkyl, ($C_3$-$C_7$)-Cycloalkyl, das substituiert sein kann, oder 2 Reste $R^4$ bilden zusammen mit dem sie verknüpfenden N-Atom einen 4- bis 7-gliedrigen heterocyclischen Ring und
n
1 bis 3
bedeuten,
durch Umsetzung einer Verbindung der Formel II

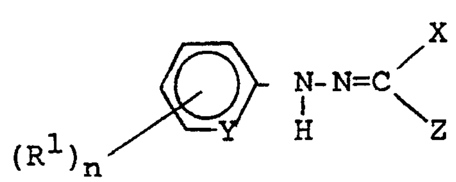

(II),

worin
$R^1$, X, Y und n die obengenannten Bedeutungen haben und
Z für Chlor oder Brom steht,
mit einem Enolether der Formel III

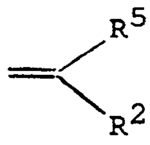

(III),

worin $R^2$ die obengenannte Bedeutung besitzt und $R^5$ = $(C_1$-$C_6)$-Alkyl, X oder $(C_1$-$C_4)$-Alkoxy ist, zu einer Verbindung der Formel IV

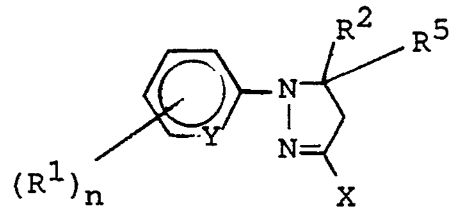

(IV),

worin die Substituenten die genannten Bedeutungen besitzen, und deren anschließender Aromatisierung zur Verbindung I, dadurch gekennzeichnet, daß man die Umsetzung der Verbindung II mit der Verbindung III zur Verbindung IV in einem 2-Phasensystem (wäßrig/organisch) in Gegenwart einer anorganischen Base durchführt und anschließend die gebildete Verbindung IV ohne vorherige Isolierung unter sauren Bedingungen in einem 2-Phasensystem (wäßrig/organisch) zur Verbindung der Formel I aromatisiert.

Alkyl bedeutet geradkettiges oder verzweigtes Alkyl.
Im Fall X =
werden zwei identishe
Reste einer Verbindung der
Formel I'miteinander verknüpft.

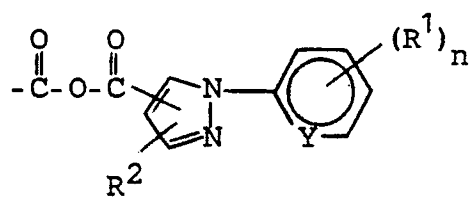

Halogen bedeutet bevorzugt Chlor oder Brom, Alkalimetall bevorzugt Li, Na, K und Erdalkalimetall insbesondere Ca. Bei dem aus den beiden Resten $R^4$ zusammen mit dem N-Atom gebildeten heterocyclischen Ring handelt es sich bevorzugt um Pyrrolidin, Morpholin, 1,2,4-Triazol und Piperidin.

Bevorzugt sind die Verbindungen der Formel I, worin Y = CH, $R^1$ = Halogen, $R^2$ = $(C_1$-$C_6)$-Alkyl, X = $COOR^3$, $R^3$ = H oder $(C_1$-$C_6)$-Alkyl und n = 1 oder 2 bedeuten.

Weiterhin bevorzugt sind die Verbindungen der Formel I, worin Y = CH, $R^1$ = C1 oder Br, $R^2$ = $(C_1$-$C_4)$-Alkyl, X = $COOR^3$, $R^3$ = $(C_1$-$C_4)$-Alkyl und n = 2 bedeuten. Insbesondere die Verbindung 1-(2,4-Dichlorphenyl)-5-isopropyl-pyrazol-3-carbonsäureethylester.

Die Verbindungen der Formel I sind bekannt. Sie finden Verwendung als Substanzen, welche die phytotoxischen Nebenwirkungen von Herbiziden wirksam reduzieren (Safener; EP-A 333 131). Verfahren zu ihrer Herstellung sind in HU-PS 153 762 und EP-A 333 131 beschrieben. Insbesondere in der Deutschen Patentanmeldung P 39 23 649.8 wird die Herstellung der Verbindungen der Formel IV ausgehend von Verbindungen der Formeln II und III und deren anschließender Aromatisierung zu den Zielverbindungen I behandelt. Dabei wird nicht in einem heterogenen 2-Phasengemisch (wäßrig/organisch) gearbeitet und die beiden Teilreaktionen werden getrennt durchgeführt, d.h. das gebildete Pyrazolin IV wird vor dessen Aromatisierung isoliert. Die zur Bildung der Verbindung IV notwendige Base, bevorzugt eine organische Base, wird dem rein organischen Reaktionsgemisch zugesetzt. Auch die zweite Teilreaktion (Aromatisierung unter sauren Bedingungen) erfolgt in einem organischen Reaktionsmedium ohne Wasserzusatz mit Hilfe einer organischen Säure. Die so erhaltene Ausbeute an den gewünschten Endprodukten beträgt über beide Stufen ca. 30 %, wodurch zusätzliche Reinigungsoperationen notwendig werden, um die Verbindungen I

von den gebildeten Nebenprodukten zu trennen. Diese Nebenprodukte müssen zusätzlich entsorgt werden. Daher ist das beschriebene Verfahren aus ökologischen als auch ökonomischen Gesichtspunkten für eine Prozeßführung im technischen Maßstab nur bedingt geeignet.

Alle diese geschilderten Nachteile vermeidet das erfindungsgemäße Verfahren, bei dem die Pyrazolcarbonsäurederivate I in Ausbeuten > 90 % d.Th. und in so hohen Reinheiten (> 95 % ) erhalten werden, daß diese Produkte ohne zusätzliche Reinigungsoperationen weiterverwendet werden können. Da eine Isolierung des Zwischenprodukts IV nicht erfolgt, ist das Verfahren aus verfahrenstechnischer Sicht besonders einfach zu handhaben. Es kann diskontinuierlich oder kontinuierlich durchgeführt werden.

Der erste Teilschritt der Reaktion (Bildung der Pyrazoline IV) wird so durchgeführt, daß man der Mischung der Ausgangsverbindungen II und III eine anorganische Base wie z.B. Alkali- oder Erdalkalihydroxide, -carbonate oder -hydrogencarbonate, insbesondere $Na_2CO_3$, $NaHCO_3$, $K_2CO_3$, $KHCO_3$, in Form ihrer wäßrigen Lösungen zusetzt. Die eintretende Cycloaddition findet somit im heterogenen 2-Phasensystem (flüssig/flüssig) statt. Dabei bilden die Verbindungen II und III die organische Phase und die Lösung der anorganischen Base die wäßrig polare Phase. Diese Reaktion im 2-Phasensystem kann mit oder ohne zusätzlichem organischem Lösungsmittel durchgeführt werden. Als organische Lösungsmittel eignen sich z.B. Toluol, Xylol oder andere mit Wasser nicht mischbare Substanzen.

Die Reaktionstemperaturen liegen bei 20-150 °C, insbesondere bei 40-100 °C, der pH-Wert der wäßrigen basischen Lösung liegt zwischen 7 und 10. Die Enolether der Formel III können äquimolar oder in größerem Überschuß (z.B. 3-facher Überschuß) eingesetzt werden und werden nach beendeter Reaktion gegebenenfalls durch Destillation recyclisiert.

Das positive Ergebnis dieses ersten Teilschritts ist als um so überraschender zu bewerten, da solche Cycloadditionen bisher nicht im 2-Phasensystem (flüssig/flüssig) beschrieben worden sind (z.B. A.S. Shawali und C. Parkänyi, J. Heterocyclic Chem. 17, 833 (1980)). Zusatz von Wasser sollte bei diesen Cycloadditionen vielmehr die Bildung von Dimerisierungsprodukten fördern (T. Shimizu et al., J. org. Chem. 1985, 50, 904-907).

Für die Bildung der Pyrazoline IV kann beim erfindungsgemäßen Verfahren auf den Zusatz eines Phasentransfer-Katalysators verzichtet werden. Unter Umständen kann der Einsatz eines solchen Katalysators jedoch zweckmäßig sein. Es handelt sich dann um die üblicherweise verwendeten Phasentransfer-Katalysatoren.

Die zweite Teilreaktion, die Aromatisierung der Verbindungen der Formel IV zu den Produkten I, wird ebenfalls im heterogenen 2-Phasengemisch durchgeführt. Hierzu wird entweder die wäßrige alkalische Phase mit Säuren (bevorzugt anorganische Säuren, wie z.B. Salz- oder Schwefelsäure) sauer gestellt (pH-Werte von 0-5, bevorzugt 0-3), oder die alkalische Phase wird abgetrennt und durch eine saure wäßrige Phase ersetzt. Die Aromatisierung zu den Produkten I geschieht in der Regel bei der Rückflußtemperatur des Reaktionsgemisches. Diese liegt zwischen 20 und 200 °C, insbesondere 50-150 °C.

Das Verhältnis von wäßriger zu organischer Phase kann innerhalb weiter Grenzen schwanken.

Ein weiterer Vorteil des erfindungsgemäßen Verfahrens liegt in der leichten Zugänglichkeit der Produkte nach beendeter Reaktion, da diese beim Abkühlen aus der 2-Phasenmischung mit über 95 % Reinheit auskristallisieren. In Abhängigkeit von der Struktur der Verbindungen I ist es zweckmäßig und häufig sogar vorteilhaft, nach beendeter Reaktion ein mit Wasser mischbares Lösungsmittel, wie z.B. Alkohole, zuzusetzen, um eine vollständige, homogene Kristallisation zu erreichen.

Die Verbindungen der Formel (II) sind zum Teil bekannt oder können nach üblichen Verfahren synthetisiert werden (EP-A 174 562). Sie lassen sich beispielsweise aus den entsprechenden Anilinen durch Diazotieren und Kuppeln mit den entsprechenden 2-Chlor-Acetessigestern erhalten. Die Verbindungen der Formel (III) sind ebenfalls nach üblichen Verfahren zugänglich, zum Beispiel durch Alkoholabspaltung aus den entsprechenden Ketalen.

Die nachfolgenden Beispiele dienen zur Erläuterung der Erfindung.

Beispiel 1

1-(2,4-Dichlorphenyl)-5-isopropyl-pyrazol-3-carbonsäureethylester

1550 g 2-Chlor-glyoxalsäureethylester-(2,4-dichlorphenylhydrazon) werden in 1000 g 2-Methoxy-3-methylbuten-1 gelöst und bei 70-80 °C mit insgesamt 600 g Kaliumhydrogencarbonat in 500 ml Wasser kontinuierlich so versetzt, daß immer ein pH-Wert von ca. 8-8,5 eingehalten wird. Nach insgesamt 4 Stunden Reaktionszeit wird der überschüssige Enolether (465 g) abdestilliert und das Reaktionsgemisch mit 190 ml Salzsäure auf einen pH-Wert von 0,5 gestellt. Nach 1-stündigem Rückflußkochen wird auf Raumtemperatur abgekühlt und vom ausgefallenen Kristallisat abgesaugt.

Es werden 1701 g Produkt mit einer Reinheit von 95,4 % erhalten, was einer Ausbeute von 95,0 %

d.Th. entspricht. Der Schmelzpunkt liegt bei 95-96 °C.

Beispiel 2
1-(2,4-Dichlorphenyl)-5-isopropyl-pyrazol-3-carbonsäureethylester

1550 g 2-Chlor-glyoxalsäureethylester-(2,4-dichlorphenylhydrazon) werden in 600 g 2-Ethoxy-3-methylbuten-1 gelöst und mit 325 g Natriumcarbonat, gelöst in 300 ml Wasser, so versetzt, daß immer ein pH-Wert größer 8,0 eingehalten wird. Nach insgesamt 4 Stunden Reaktionszeit (60 - 80 °C) wird die wäßrige Phase abgetrennt und die organische Phase mit 200 ml Salzsäure versetzt (pH~1) und 1 Stunde zum Rückfluß erhitzt. Nach Abkühlen auf 70 °C wird die organische Phase abgetrennt und mit Methanol/Wasser zur Kristallisation gebracht. Es werden 1694 g Produkt mit einer Reinheit von 96,3 % erhalten, was einer Ausbeute von 94,7 % d.Th. entspricht. Der Schmelzpunkt liegt bei 95-96 °C.

Analog können die Beispiele der Tabelle I hergestellt werden.

Tabelle I Pyrazole I

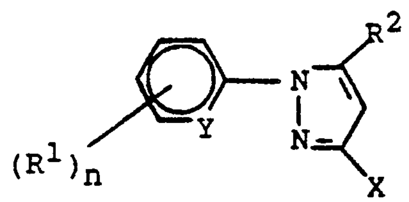

(I)

| Bsp.-Nr. | X | $(R^1)_n$ | $R^2$ | Y | Ausbeute |
|---|---|---|---|---|---|
| 3 | $CO_2C_2H_5$ | 2,4-$Cl_2$ | $C_2H_5$ | CH | 93,2 % |
| 4 | $CO_2CH_3$ | 2,4-$Cl_2$ | $CH(CH_3)_2$ | CH | 92,7 % |
| 5 | $CO_2(CH_2)_2OCH_3$ | 2,4-$Cl_2$ | $CH(CH_3)_2$ | CH | 94,4 % |
| 6 | $CO_2C_2H_5$ | 2,4-$Cl_2$ | $C_2H_3$ | CH | 95,1 % |
| 7 | $CO_2C_2H_5$ | 2,4-$Cl_2$ | $-C\equiv CH$ | CH | 91,2 % |
| 8 | $CO_2C_2H_5$ | 2,4-$CF_3$-Cl | $CH(CH_3)_2$ | CH | 93,1 % |
| 9 | $CO_2C_2H_5$ | 2,4-$Cl_2$ | $C_4H_9$ | CH | 92,3 % |

**Patentansprüche**

1. Verfahren zur Herstellung von Pyrazolcarbonsäurederivaten der Formel I

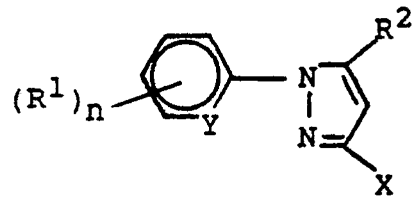

(I),

worin

Y

C-H oder N,

$R^1$

unabhängig voneinander ($C_1$-$C_4$)-Alkyl, ($C_1$-$C_4$)-Haloalkyl, ($C_1$-$C_4$)-Alkoxy, ($C_1$-$C_4$)-Haloalkoxy oder Halogen,

$R^2$

($C_1$-$C_{12}$)-Alkyl, ($C_3$-$C_7$)-Cycloalkyl, ($C_2$-$C_6$)-Alkenyl oder ($C_2$-$C_4$)-Alkinyl,

X

$COOR^3$, $CON(R^4)_2$, $COSR^3$, CN,

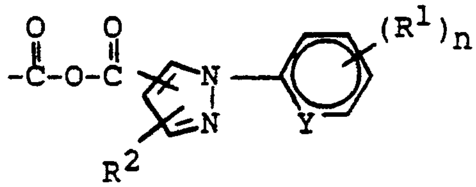

,

$R^3$

Alkali- oder Erdalkalimetall, Wasserstoff, ($C_1$-$C_{10}$)-Alkyl, ($C_3$-$C_{20}$)-Alkenyl, ($C_3$-$C_{10}$)-Alkinyl, ($C_3$-$C_7$)-Cycloalkyl, Phenyl-($C_1$-$C_4$)-Alkyl, wobei Phenyl durch Halogen substituiert sein kann, Tris-($C_1$-$C_4$)-Alkyl-Silyl-($C_1$-$C_4$)-alkyl, ($C_1$-$C_4$)-Alkoxy-($C_1$-$C_4$)-alkyl

$R^4$

unabhängig voneinander H, ($C_1$-$C_{10}$)-Alkyl, ($C_3$-$C_7$)-Cycloalkyl, das substituiert sein kann, oder 2 Reste $R^4$ bilden zusammen mit dem sie verknüpfenden N-Atom einen 4- bis 7-gliedrigen heterocyclischen Ring und

n

1 bis 3

bedeuten,

durch Umsetzung einer Verbindung der Formel II

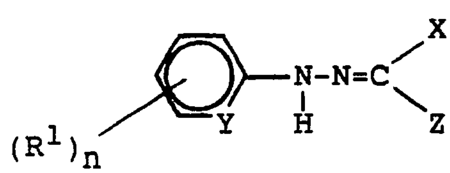

(II),

worin

$R^1$, X, Y, und n die obengenannten Bedeutungen haben und

Z für Chlor oder Brom steht,

mit einem Enolether der Formel III

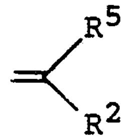

(III),

worin $R^2$ die obengenannte Bedeutung besitzt und $R^5$ = $(C_1$-$C_6)$-Alkyl, X oder $(C_1$-$C_4)$Alkoxy ist, zu einer Verbindung der Formel IV

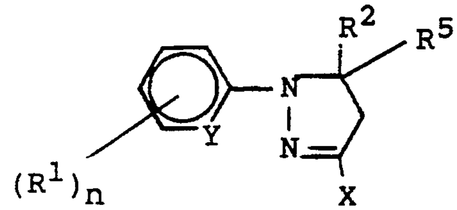

(IV),

worin die Substituenten die genannten Bedeutungen besitzen, und deren anschließender Aromatisierung zur Verbindung I, dadurch gekennzeichnet, daß man die Umsetzung der Verbindung II mit der Verbindung III zur Verbindung IV in einem 2-Phasensystem (wäßrig/organisch) in Gegenwart einer anorganischen Base durchführt und anschließend die gebildete Verbindung IV ohne vorherige Isolierung unter sauren Bedingungen in einem 2-Phasensystem (wäßrig/organisch) zur Verbindung der Formel I aromatisiert.

2. Verfahren gemäß Anspruch 1, dadurch gekennzeichnet, daß in Formel I Y = CH, $R^1$ = Cl oder Br, $R^2$ = $(C_1$-$C_4)$-Alkyl, X = $COOR^3$, $R^3$ = $(C_1$-$C_4)$-Alkyl und n = 2 bedeuten.

3. Verfahren gemäß Anspruch 1 oder 2, dadurch gekennzeichnet, daß es sich bei der Verbindung der Formel I um 1-(2,4-Dichlorphenyl)-5-isopropyl-pyrazol-3-carbonsäureethylesterhandelt.

4. Verfahren gemäß einem oder mehreren der Ansprüche 1-3, dadurch gekennzeichnet, daß man die Umsetzung der Verbindung II mit der Verbindung III zum Zwischenprodukt IV bei Temperaturen von 40-100 °C und bei pH-Werten von 7-10 durchführt.

5. Verfahren gemäß einem oder mehreren der Ansprüche 1-4, dadurch gekennzeichnet, daß man bei der Umsetzung der Verbindung II mit der Verbindung III zum Zwischenprodukt IV als anorganische Base eine wäßrige Lösung von $Na_2CO_3$,$NaHCO_3$, $K_2CO_3$ oder $KHCO_3$ verwendet.

6. Verfahren gemäß einem oder mehreren der Ansprüche 1-5, dadurch gekennzeichnet, daß man die Umsetzung des Zwischenprodukts IV zur Verbindung I bei pH-Werten von 0-3 und der Rückflußtemperatur des Reaktionsgemisches durchführt.